# EUROPEAN PATENT APPLICATION

(11) **EP 1 658 864 A2**
(43) Date of publication of application: **24.05.2006**
(21) Application number: 05025171.9
(22) Date of filing: 17.11.2005
(51) Int. Cl.: A61L 9/20, H01J 61/35, H01J 61/30, H01J 61/72

(54) **Quartz gas discharge lamp providing photocatalytic oxidation**

(30) Priority: 23.11.2004 US 995649
(71) Applicant: Light Sources, Inc., Orange, CT 06477 (US)
(72) Inventor: Ward, Patrick, Connecticut 06516 (US); Gavagan, Brian, Southport Connecticut 06890 (US)
(74) Representative: Grünecker, Kinkeldey, Stockmair & Schwanhäusser Anwaltssozietät

(57) **Abstract**

A germicidal quartz lamp (10; 110; 210) producing ultraviolet radiation having a sleeve coated (20; 120; 220) with a titanium dioxide (18; 118A, 118B; 218). Titanium dioxide (18; 118A, 118B; 218) is impregnated or embedded into the sleeve (20; 120; 220) and mechanically held. Ultraviolet radiation having a wavelength of approximately 254 nm generated by the germicidal quartz lamp causes hydroxyl radicals to form on the titanium dioxide surface 18; 118A, 118B; 218). The hydroxyl radicals act as a powerful oxidizing agent that destroys pollutants. In one embodiment, different densities of the titanium dioxide (118A, 118B) coating are formed along the longitudinal length of the quartz lamp. A longitudinal portion (118C) of the quartz lamp may be free of any titanium dioxide coating permitting the ultraviolet radiation to pass therethrough creating a germicidal effect. The titanium dioxide may be heated to a temperature above the melting point of the sleeve to create the mechanical bond. The present invention is useful in the commercial food industry or for consumer use.

## Description

### FIELD OF THE INVENTION

The present invention relates in general to remediation of pollutants with an oxidation agent and more particularly to a gas discharge quartz lamp providing ultraviolet radiation for reacting with a coating, resulting in photocatalytic oxidation.

### BACKGROUND OF THE INVENTION

It is known that the remediation of pollutants, in particular volatile organic compounds, may be accomplished using photocatalytic oxidation or advanced oxidation technology, known as AOT. Typically, advanced oxidation technology involves the irradiation of an anatase form of titanium dioxide or titania by ultraviolet radiation having a wavelength below 385 nm. Hydroxyl radicals formed in the irradiated titania act as a powerful oxidizing agent that destroys pollutants.

In many industrial applications, such as in the remediation of contaminated ground water, quartz gas discharge lamps that produce ultraviolet radiation having a wavelength of substantially 254 nm are used to irradiate flat beds coating with titania. The contaminated ground water flows over the coated flat beds.

In another application of the advanced oxidation technology, a reaction vessel is filled with titania pellets through which contaminated water flows and which are irradiated by ultraviolet quartz lamps.

There is much interest in providing the remediation of pollutants with an oxidizing agent for smaller scale commercial and home use. Efforts to accomplish this goal have been directed to applying a coating directly to fluorescent lamps. However, the use of fluorescent lamps is generally not desirable because of their inefficient and relatively short life due to degradation of the ultraviolet phospors and their limited or low power output. The use of higher power, longer life ultraviolet gas discharge lamps made from quartz would be desirable, however additional problems with the use of quartz arise. For example, quartz is a difficult material to work with and it is extremely difficult to coat or have material adhere to a quartz surface.

Accordingly, there is a need for a relatively compact, self contained lamp capable of producing photocatalytic oxidation forming hydroxyl radicals for use as an oxidizing agent to destroy pollutants.

### SUMMARY OF THE INVENTION

The present invention comprises a tubular quartz gas discharge lamp producing ultraviolet radiation and having an embedding material or a sleeve with a coating of titanium dioxide thereon. The embedding material or sleeve is preferably made of FEP Teflon or fluorinated ethylene propylene shrink tubing, or other suitable fluoropolymer material.

In an embodiment of the invention, the sleeve is coated along its longitudinal extent with predetermined different segments with different coating density or thicknesses. This permits ultraviolet radiation to pass through so as to add a germicidal effect, due to the ultraviolet radiation, in a single lamp. Another embodiment of the invention relates to a method of making a quartz gas discharge lamp having photocatalytic oxidation properties comprising the steps of heating a titania powder to a temperature greater than the melting point of a sleeve and rolling the sleeve in the heated titania powder for a predetermined time and pressure so that the titania powder adheres to the sleeve, with the titania powder having a predetermined thickness.

Accordingly, it is an object of the present invention to utilize a quartz gas discharge lamp with a photocatalytic oxidation coating.

It is a further object of the present invention to combine remediation of pollutants and germicidal action in a single lamp.

It is an advantage of the present invention that, in the event of breakage of the quartz lamp envelope, the quartz glass and mercury is contained within the sleeve, resulting in safer operations of the lamp.

It is a further advantage of the present invention that the proportion or the degree of remediation of pollution can be easily controlled.

It is a feature of the present invention that a sleeve is impregnated with a titanium dioxide and placed over a quartz gas discharge lamp.

It is another feature of the present invention that longitudinal segments along the length of the quartz envelope of the gas discharge lamp are coating with a selected density of titanium dioxide based on a particular application.

These and other objects, advantages, and features will become more readily apparent in view of the following, more detailed description.

### BRIEF DESCRIPTION OF THE DRAWINGS

Fig. 1 is a perspective view illustrating an embodiment of the present invention.
Fig. 2 is a cross section taken along line 2-2 in Fig. 1.
Fig. 3 is a cross section taken along line 3-3 in Fig. 2.
Fig. 4 is a perspective view of another embodiment of the present invention.
Fig. 5 is a partial exploded view illustrating an alternative structure for the end of a quartz ultraviolet gas discharge lamp.
Fig. 6 is a schematic view illustrating the application of the present invention in a room.
Fig. 7 is a block diagram illustrating the method steps of an embodiment of the present invention.

### DETAILED DESCRIPTION OF THE PREFERRED EMBODIMENTS

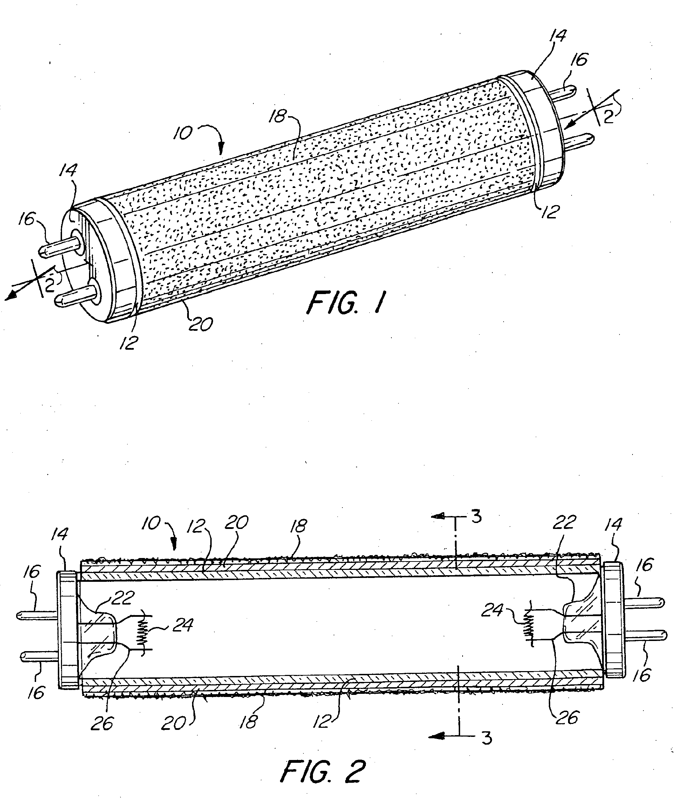

Fig. 1 is a perspective view schematically illustrating an embodiment of the present invention. A gas discharge lamp 10 having photocatalytic oxidation properties is illustrated. The gas discharge lamp 10 has a quartz tube 12 with end caps 14. End caps 14 have contact pins 16 extending therefrom. Placed around the quartz tube 12 is a sleeve 20. Impregnated on the sleeve 20 is titanium dioxide 18. The sleeve 20 is preferably made of a material that has a melting point lower than the quartz tube 12 and the titanium dioxide 18. The sleeve 20 is preferably made of fluorinated ethylene propylene or other suitable fluropolymer commonly sold under the trademark Teflon. The fluorinated ethylene propylene sleeve is durable and can withstand relatively severe conditions. The fluorinated ethylene propylene sleeve generally has non-adhesive qualities, however, when it is in a plastic state approaching its melting point of approximately 225°C, the titanium dioxide 18 material may be impregnated or embedded therein and mechanically held. The titanium dioxide 18 impregnated or embedded on the sleeve 20 is preferably an anatase form of titanium dioxide, also referred to as titania. Anatase is a white tetragonal crystalline form of titanium dioxide, which is also known as actahedrite and is sometimes used to make a white pigment among other applications.

When the anatase is irradiated with ultraviolet radiation having a wavelength below approximately 385 nm, electron and hole pairs are formed on the surface. In the presence of water vapor, hydroxyl radicals are formed on the titania surface. The hydroxyl radicals act as a powerful oxidizing agent that destroys pollutants without the release of any harmful agents, such as ozone, entering the environment. The fluorinated ethylene propylene sleeve 20 transmits a substantial quantity, at least eighty percent, of the ultraviolet radiation generated by the quartz gas discharge lamp.

Fig. 2 is a longitudinal cross section taken along line 2-2 in Fig. 1. Fig. 2 more clearly illustrates the structure of the quartz gas discharge lamp 10 having the photocatalytic oxidation properties. Fig. 2 illustrates the stem or support 22 used to support lead wires 26 attached to an electrode or filament 24. The lead wires 26 are electrically coupled to contact pins 16. A gas, including a mercury vapor, contained within the quartz tube 12 will become energized upon application of a voltage between the electrodes 24. The resulting arc or ionized gases will cause ultraviolet radiation to be generated. Quartz gas discharge lamps of this nature,are commonly used in germicidal applications. Generally, ultraviolet radiation having a wavelength of approximately 254 nm is generated. As is clearly seen in Fig. 2, the quartz tube 12 is covered by the fluorinated ethylene propylene sleeve 20 which is impregnated with a coating of titanium dioxide or titania 18.

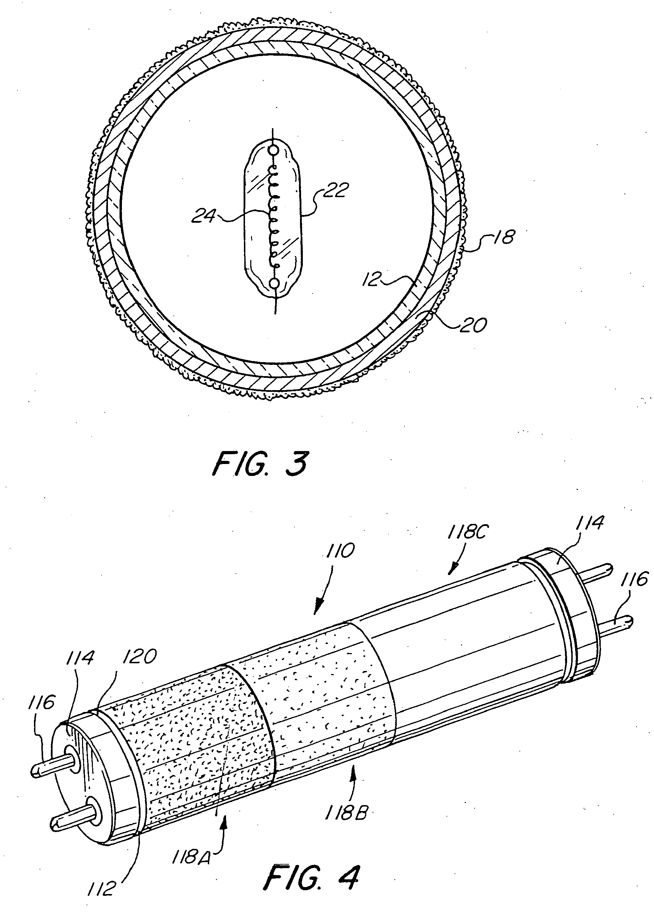

Fig. 3 is a lateral cross section taken along line 3-3 in Fig. 2. The stem or support 22 may be a separate piece or is preferably pressed from the quartz tube 12 so as to form a seal and support for the lead wires 26, as is well known and conventional in the manufacture of quartz gas discharge lamps.

Fig. 4 schematically illustrates a perspective view of another embodiment of the invention having different segments along the longitudinal length with each different segment having varying degrees of coatings placed on the sleeve. The titanium dioxide material may have different densities spatially in different areas of the lamp. Similar to the prior embodiments, a quartz tube 112 has end caps 114 thereon with contact pins 116. A fluorinated ethylene propylene sleeve 120 is placed over the longitudinal length of the quartz tube 112. Each longitudinal segment of the fluorinated ethylene propylene sleeve 120 is coated with a different quantity of titania or titanium dioxide. For example, longitudinal section 118A may have a relatively heavy coating of titania or titanium dioxide. Longitudinal section 118B may have a coating of titania or titanium dioxide that is less dense than that of the coating along section 118A. Longitudinal section 118C may be free from any coating, permitting the ultraviolet radiation generated by the lamp 110 to be transmitted therethrough providing a germicidal effect. Therefore, in this embodiment the lamp 110 has a germicidal effect together with the pollution reduction effects of the hydroxyl radicals formed by the interaction of the ultraviolet radiation with the titania or titanium dioxide.

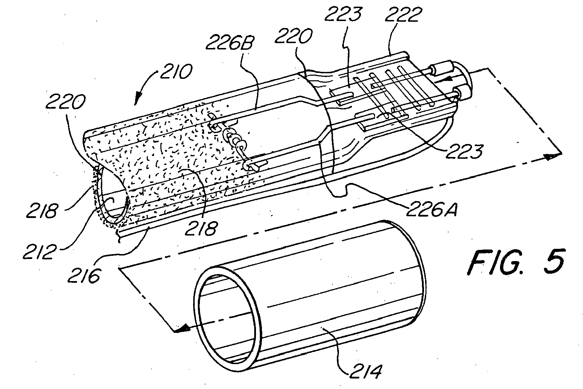

Fig. 5 is an exploded perspective view of a portion of a lamp having a different pressed end construction. A photocatalytic oxidation lamp 210 comprises a quartz tube 212 having a pressed stem 222. Metal ribbon contacts 223 are pressed within the stem 222 and are electrically connected to lead wires 226A and 226B that support electrode or filament 224. Wire 216 is used to make electrical contact with a pin at the other end of the lamp 210, not shown. End cap 214 covers the pressed stem 222. A fluorinated ethylene propylene sleeve 220 covers the quartz tube 212. Impregnated or embedded on the fluorinated ethylene propylene sleeve 220 is titania or titanium dioxide 218.

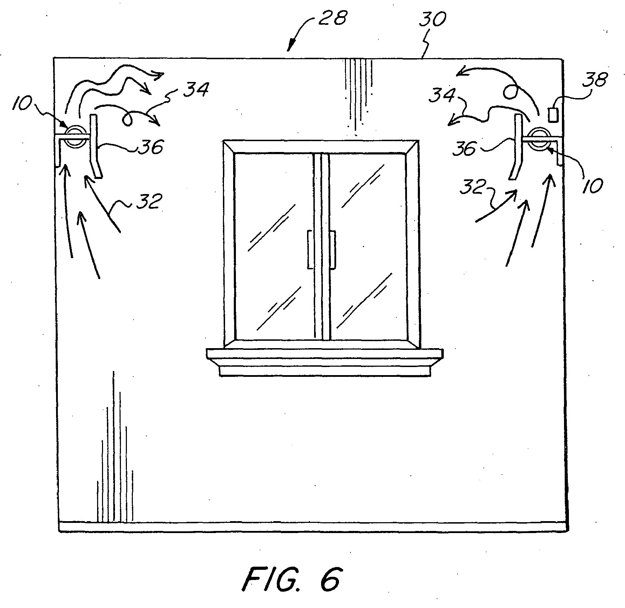

Fig. 6 schematically illustrates the application of the present invention for removing pollutants from a room. An air purifying system 28 may be utilized in a room 30 having a plurality of photocatalytic oxidation gas discharge lamps 10 placed therein. The lamp 10 may be placed behind a shield 36. Arrows 32 represent air flow that may be contaminated with pollutants that are directed over the lamp 10. Arrows 34 represent the output air flow that has been purified or decontaminated. A fan 38 may be used to augment the air flow over the lamp 10. The lamp 10 may have a portion thereon that is not coated with the titanium dioxide, permitting the ultraviolet radiation generated by lamp 10 to be transmitted therethrough, providing a germicidal action. Therefore, by selectively coating the longitudinal length or other portions of the lamp 10, pollutants from the air can be remediated or removed, and in addition the destruction of other organisms that may result in illness can be achieved as a result of the germicidal action of the ultraviolet radiation.

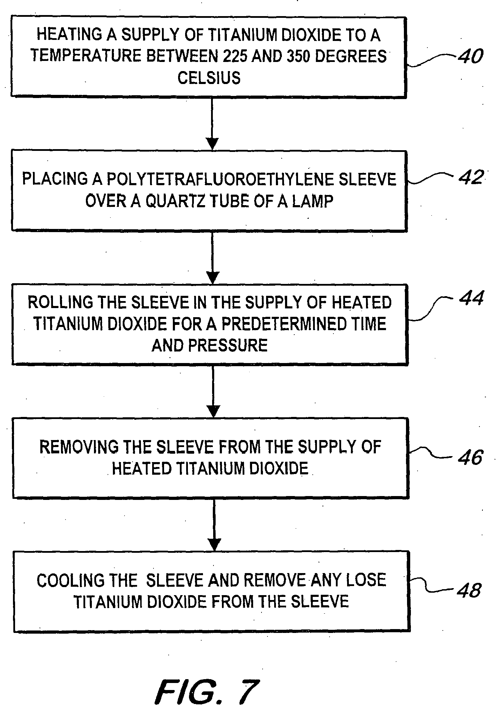

Fig. 7 is a block diagram illustrating the method steps of an embodiment of the present invention. Block 40 represents the method step of heating a supply of titanium dioxide to a temperature between 225° and 350°C. Block 42 represents the method step or act of placing a fluorinated ethylene propylene sleeve over a quartz tube of a lamp. Block 44 represents the method steps or acts of rolling the sleeve in a supply of heated titanium dioxide with a predetermined pressure for a predetermined time so as to permit the titanium dioxide to become embedded in the fluorinated ethylene propylene sleeve. Block 46 represents the step of removing the sleeve from the supply of heated titanium dioxide. Block 48 represents the method step of cooling the sleeve and removing any loose titanium dioxide from the sleeve. After these method steps or acts, a titanium dioxide coating will be mechanically impregnated or embedded on the fluorinated ethylene propylene sleeve.

Accordingly, since the titanium dioxide is heated to a temperature above the melting point of the fluorinated ethylene propylene sleeve, upon contact the sleeve will deform and permit the titanium dioxide particles to become embedded therein. Therefore, a mechanical bond is formed on the sleeve made of a fluorinated ethylene propylene material. This is contrary to what would be expected of a fluorinated ethylene propylene material, because generally a fluorinated ethylene propylene material is considered to have a non-stick surface.

Accordingly, the present invention makes possible the application of a titanium dioxide coating to a quartz germicidal lamp. The quartz germicidal lamp surface typically is a surface that is difficult to apply a coating to. The fluorinated ethylene propylene sleeve is capable of surviving the high temperatures of the quartz lamp during operation, as well as acts as a safety barrier in the event of breakage of the quartz glass tube so as to contain any glass or mercury or other contaminants. The present invention, therefore, is particularly applicable to commercial use, such as in the food service business or in the home environment where safety is of primary concern. Therefore, the present invention makes applicable an easy to install and maintain photocatalytic oxidation lamp that may also have a germicidal effect that can be used in many applications in which prior, larger scale industrial applications using titanium dioxide could not be utilized.

While the present invention has been illustrated and described with respect to several embodiments, it will be obvious to those skilled in the art that variations or modifications may be made without departing from the spirit and scope of this invention.

## Claims

1. A lamp used for the remediation of pollutants comprising:
a source of ultraviolet radiation within a glass tube;
a sleeve placed over at least a portion of the glass tube; and
a photocatalytic material embedded in said sleeve,
whereby upon operation of the lamp, ultraviolet radiation striking said photocatalytic material forms an oxidizing agent that remediates pollution.

2. A lamp used for the remediation of pollutants as in claim 1 wherein:
the glass tube comprises quartz glass.

3. A lamp used for the remediation of pollutants as in claim 1 wherein:
said sleeve comprises a fluoropolymer.

4. A lamp used for the remediation of pollutants as in claim 1 wherein:
said photocatalytic material comprises titanium dioxide.

5. A lamp used for the remediation of pollutants as in claim 4 wherein:
said titanium dioxide comprises an anatase form of titanium dioxide.

6. A lamp used for the remediation of pollutants as in claim 1 wherein:
said photocatalytic material is imbedded in only a portion of said sleeve,
whereby a portion of the ultraviolet radiation from said source passes though without striking said photocatalytic material resulting in an additional germicidal effect.

7. A device for the remediation of pollutants comprising:
an ultraviolet lamp;
a sleeve placed over said ultraviolet lamp; and
a photocatalytic material embedded in said sleeve,
whereby upon operation of the lamp, ultraviolet radiation striking said photocatalytic material forms an oxidizing agent that remediates pollution.

8. A device for the remediation of pollutants as in claim 7 wherein:
said sleeve comprises a fluoropolymer.

9. A device for the remediation of pollutants as in claim 8 wherein:
the fluropolymer comprises polytetrafluooethylene.

10. A device for the remediation of pollutants as in claim 7 wherein:
said photocatalytic material comprises titanium dioxide.

11. A device for the remediation of pollutants as in claim 10 wherein:
said titanium dioxide comprises an anatase form of titanium dioxide.

12. A pollutant remediation device comprising:
an ultraviolet lamp;
an embedding material placed over said ultraviolet lamp; and
a photocatalytic material embedded in said embedding material,
whereby upon operation of the lamp, ultraviolet radiation striking said photocatalytic material forms an oxidizing agent that remediates pollution.

13. A pollutant remediation device as in claim 12 wherein:
said embedding material has a melting point less than said photocatalytic material.

14. A pollutant remediation device as in claim 12 wherein:
said embedding material comprises a fluoropolymer.

15. A pollutant remediation device as in claim 14 wherein:
the fluoropolymer comprises fluorinated ethylene propylene.

16. A pollutant remediation device as in claim 12 wherein:
said photocatalytic material comprises titanium dioxide.

17. A pollutant remediation and germicidal device comprising:
an ultraviolet lamp capable of generating ultraviolet radiation;
an embedding material covering a first portion of said ultraviolet lamp; and
a photocatalytic material embedded in at least a portion of said embedding material,
whereby said photocatalytic material receives a portion of the ultraviolet radiation generated from said ultraviolet lamp and another portion of the ultraviolet radiation generated from said ultraviolet lamp is emitted from the pollutant remediation and germicidal device providing a combined pollutant remediation and germicidal effect.

18. A pollutant remediation and germicidal device as in claim 17 wherein:
said photocatalytic material has a density that varies spatially on said embedding material.

19. A pollutant remediation and germicidal device as in claim 17 wherein:
said embedding material comprises a fluoropolymer.

20. A method of making a photocatalytic lamp comprising the steps of:
placing an embedding material over an ultraviolet lamp; and
embedding a photocatalytic material into the embedding material,
whereby upon operation of the ultraviolet lamp, ultraviolet radiation striking the photocatalytic material forms an oxidizing agent that remediates pollution.

21. A method of making a photocatalytic lamp as in claim 20 wherein:
the embedding material comprises a fluoropolymer.

22. A method of making a photocatalytic lamp as in claim 20 wherein:
the photocatalytic material comprises titanium dioxide.

23. A method of making a lamp promoting photocatalytic oxidation comprising the steps of:
heating a photocatalytic material to a temperature equal to or greater than a melting point of an embedding material;
placing the embedding material on an ultraviolet lamp; and
rolling the embedding material in the photocatalytic material,
whereby the photocatalytic material locally melts the embedding material embedding the photocatalytic material in the embedding material.

24. A method of making a lamp promoting photocatalytic oxidation as in claim 23 further comprising the step of:
applying a predetermend pressure to the embedding material during the step of rolling the embedding material in the photocatalytic material.

25. A method of making a lamp promoting photocatalytic oxidation as in claim 23 wherein:
the embedding material comprises a fluoropolymer.

26. A method of making a lamp capable of photocatalytic oxidation comprising the steps of:
heating titanium dioxide particles to a temperature between 225°C and 350°C;
placing a fluorinated ethylene propylene sleeve on a tubular ultraviolet lamp;
rolling the fluorinated ethylene propylene sleeve on the tubular ultraviolet lamp in the titanium dioxide particles heated to a temperature between 225°C and 350°C while applying a predetermined pressure on the tubular ultraviolet lamp;
cooling the fluorinated ethylene propylene sleeve; and
removing unattached titanium dioxide particles from the fluorinated ethylene propylene sleeve,
whereby the titanium dioxide particles locally melt the fluorinated ethylene propylene sleeve embedding the titanium dioxide particles in the fluorinated ethylene propylene sleeve so as to be mechanically held therein.
